# EUROPEAN PATENT SPECIFICATION

(11) **EP 1 783 123 B1**
(45) Date of publication and mention of the grant of the patent: **17.03.2010**
(21) Application number: 05769869.8
(22) Date of filing: 20.06.2005
(51) Int. Cl.: C07D 319/06, C07D 493/18, A61K 31/357

(54) **SYNTHESIS OF POLYOXYGENATED NITROGEN SYSTEMS, COMPRISING REACTIONS BETWEEN ENAMINES OF 1,3-DIOXAN-5-ONES AND NITROOLEFINS**
SYNTHESE VON POLYOXYGENIERTEN STICKSTOFFSYSTEMEN, BEI DER MAN ENAMINE VON 1,3-DIOXAN-5-ONEN UND NITROOLEFINE MITEINANDER UMSETZT
SYNTHESE DE SYSTEMES AZOTES POLYOXYGENES MOYENNANT DES REACTIONS ENTRE DES ENAMINES DE 1,3-DIOXAN-5-ONES ET DES NITROOLEFINES

(30) Priority: 25.06.2004 ES 200401547
(43) Date of publication of application: 09.05.2007
(73) Proprietor: UNIVERSIDADE DE SANTIAGO DE COMPOSTELA, 15782 Santiago de Compostela (ES)
(72) Inventor: ALONSO ALONSO, R., Uni. de Santiago de Compostela, E-15782 Santiago de Compostela (ES); OZORES VITURRO, L., Uni. de Santiago de Compostela, E-15782 Santiago de Compostela (ES); CAGIDE FAGÍN, F., Uni. de Santiago de Compostela, E-15782 Santiago de Compostela (ES); ORTIZ LARA, J. C., Uni. de Santiago de Compostela, E-15782 Santiago de Compostela (ES)
(74) Representative: ABG Patentes, S.L.
(86) International application number: PCT/ES2005/000374
(87) International publication number: WO 2006/003227

(56) References cited:
- WO-A2-97/16453
- SEEBACH D. ET AL: 'Synthesis of open-chain, 2,3-disubstituted-4-nitroketones by diastereoselective Michael-addition of (E)-enamines to (E)-nitroolefins. A topological rule for C,C-bond forming processes between prochiral centres' HELVETICA CHIMICA ACTA vol. 64, no. 5, 1981, pages 1413 - 1423, XP003009246
- MARCH J.: 'Advanced Organic Chemistry. Reactions, Mechanisms and Structure', 1992, JOHN & WILEY & SONS page 1168, XP008077699
- ORTIZ J.C. ET AL.: "Annulation of beta-aryl-alpha-nitro-alpha,beta-enals and 2,2-dimethyl-1,3-dioxan-5- one: a one-step assembly of nitrocyclitols. Application to a short practical synthesis of (+-)-7-deoxy-2-epi-pancratistatin tetraacetate" CHEMICAL COMMUNICATIONS, 2006, pages 4239-4241,

## Description

### Field of the Invention

The invention is included in the general area of development of new processes for the stereocontrolled synthesis of polyoxygenated nitrogen systems and particularly in the area of development of new synthetic strategies for the preparation of those compounds having polyoxygenated nitrogen cyclohexane systems as a structural subunit, as is the case of: a) certain antibiotics such as streptomycin, kanamycins, fortimicins or hygromycin A, b) certain compounds blocking voltage-dependent sodium channels such as tetrodotoxin and the analogues thereof, and c) certain compounds with a proven antitumor and antiviral efficacy such as pancratistatin and the analogues thereof.

### State of the Art

Polyoxygenated nitrogen cyclohexane systems constitute an essential part of the structures of a high number of compounds of great biological and pharmacological interest. These compounds include:
1. Certain antibiotics such as streptomycin, kanamycins, fortimicins or hygromycin A: 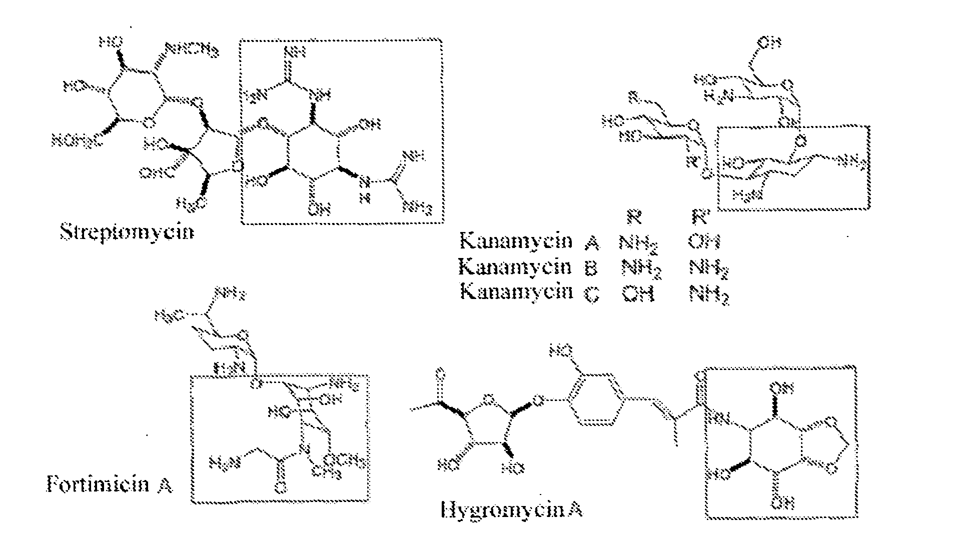
2. Certain compounds blocking voltage-dependent sodium channels (and therefore blocking nervous impulse and pain sensation) such as tetrodotoxin and the analogues thereof: 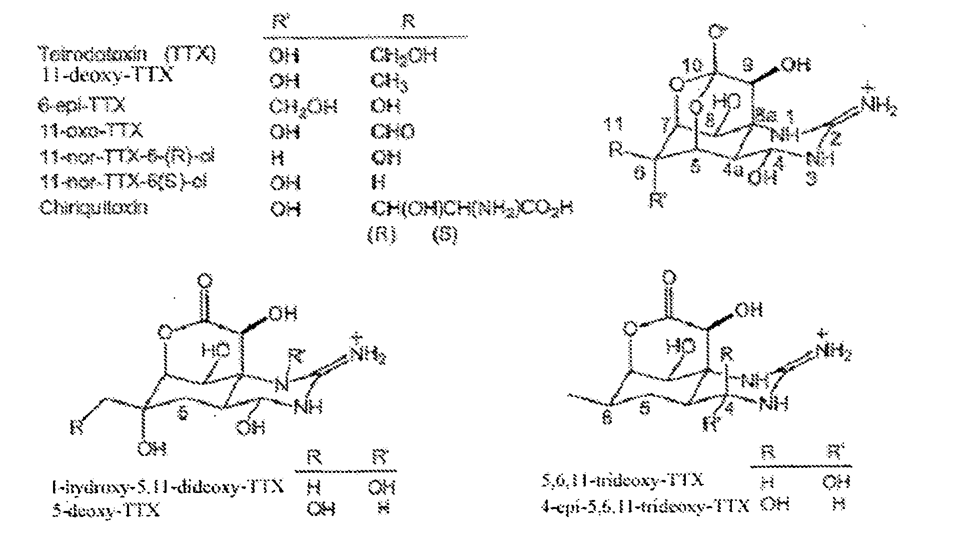
and 3. Certain compounds with a proven antitumor and antiviral efficacy such as pancratistatin and the analogues thereof.

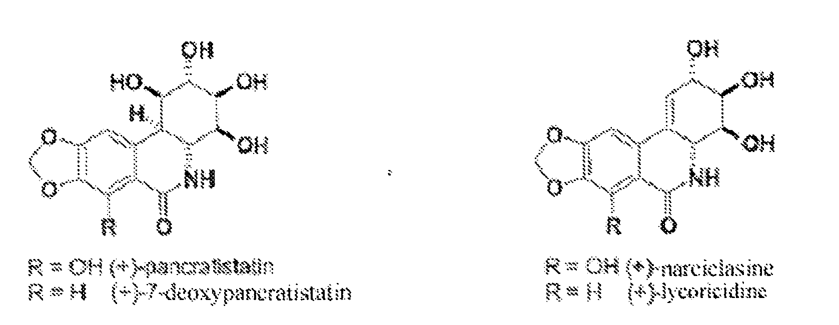

In spite of the importance of the mentioned polyoxygenated nitrogen systems derived from their presence as structural subunits in compounds of great interest, as illustrated above, the efficient access to said systems is not currently solved. By way of example, the following facts are enough to demonstrate this:
1. The synthesis of the polyhydroxylated aminocyclohexane of the antibiotic hygromycin A recently described by Trost (Trost, B. M.; Dudash, J., Jr.; Hembre, E.J., Chem. Eur. J. 2001, 7, 1619-1629) still requires a total of 13 steps, despite the fact that it involves an important improvement compared to the only synthesis described previously (Chida, N.; Ohtsuka, M.; Nakazawa, K.; Ogawa, S., J. Org. Chem. 1991, 56, 2976-2983).
2. The total synthesis of tetrodotoxin in an enantiomerically pure form has just been achieved by two groups (Hinman, A.; Du Bois, J., J. Am. Chem. Soc. 2003, 125, 11510-11511, Ohyabu, N.; Nishikawa, T.; Isobe, M., J. Am Chem. Soc. 2003, 125, 8798-8805) and in both cases requires a considerably high number of steps, more than 30 in the first case and more than 70 in the second.
3. Although up to eight total syntheses of pancratistatin have currently been described, none of them has solved the problem for supplying this compound, a problem which has determined the standstill of preclinical studies which were being conducted in the U.S. National Cancer Institute (NCI) (see the last total syntheses described by S. Kim et al.: Ko, H.; Kim, E.; Park, J.E.; Kim, D.; Kim, S., J. Org. Chem. 2004, 69, 112-121, as well as the synthesis studies by Kornienko et al.: Nadein, O.N.; Kornienko, A. Org. Lett. 2004, 6, 831-834 and the references mentioned therein) and WO96/25415.

From the foregoing, it is obvious that there is a need to invent new, general synthetic strategies allowing access to polyoxygenated nitrogen systems, particularly cyclohexane systems, in a small number of steps.

This patent sets forth a solution allowing the expeditious access to polyoxygenated nitrogen systems starting from simple and easily obtainable compounds, in a convergent manner and in a very reduced number of steps.

### Description of the Invention

The present invention describes the formation of polyoxygenated nitrogen systems by means of the reaction of enamines having formula II with nitroolefins having formula III.

Enamines II can be prepared by the reaction of ketones I, which obviously allow an extraordinarily varied substitution for the indicated substituents R¹-R⁴, with nitrogen compounds having formula R⁵R⁶NH, wherein R⁵ and R⁶ can also have extraordinarily different values (scheme 1, example 1). The reaction of enamines II with nitroolefins III, where the only possible structural limitation could be that in which any of the substituents R⁷ or R⁸ introduces a double bond conjugated with the one in the nitroolefin, leads to polyoxygenated nitrogen compounds having formula IV by Michael-type addition (Scheme 1, example 2). For a general process for the Michael-addition of enamines to nitroolefines see Seebach, D. et al., Helvetica Chimica Acta, 1981, 64, 1413-1423. 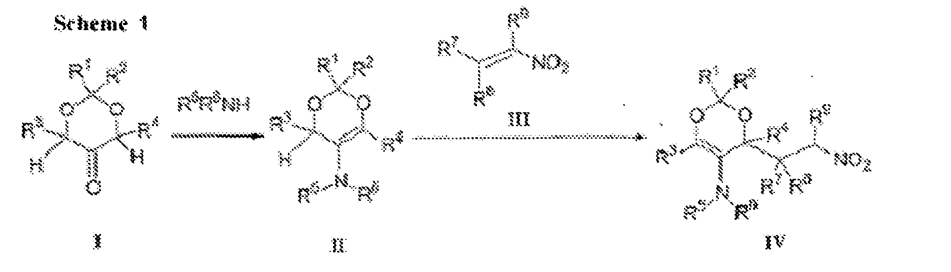 When nitroolefins III have:
i) either an electrophilic R⁹ group which can be attacked by an enamine, such as III wherein R⁹ = C(O)X wherein X can be a hydrogen or a halogen atom or O, S, Se or Te atoms joined to an alkyl, aryl or acyl group, or
ii) an electrophilic center precursor R⁹ group such as III wherein R⁹ = CXYZ, wherein X and Y can be hydrogen or halogen atoms or alkyl or aryl groups which can in turn be substituted by one or several identical or different substituents selected from halogen atoms, alkyl, aryl, hydroxy, alkoxy, aryloxy, amino, alkylamino or arylamino groups, and X is any leaving group such as: a) a halogen atom, or b) an -OC(O)R^{b} group, or c) an -OS(O)ₙR^{b} wherein n can have the values 1 or 2, and R^{b} can be a linear, branched or cyclic alkyl group or an aryl group, which groups can in turn be substituted by one or several identical or different substituents selected from halogen atoms, alkyl, hydroxy, alkoxy, aryloxy, amino or alkylamino groups, then the reaction of these nitroolefins III with enamines II does not stop in compound IV, but the enamino group present in IV reacts with the acceptor group in R⁹ to form the intermediates A which lead to bicyclic polyoxygenated nitrogen compounds having formula V by hydrolysis (scheme 2, examples 3, 4 and 5).

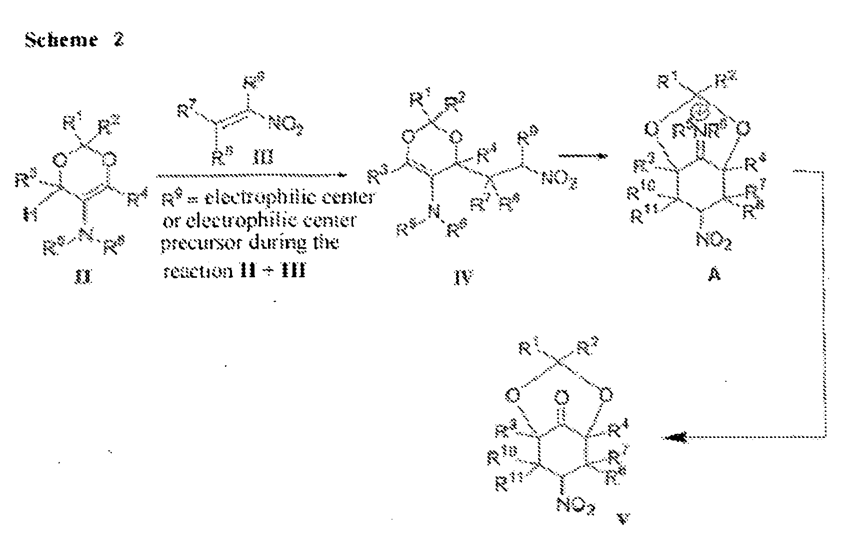

Alternatively, bicycles V can be obtained starting from compounds having formula IV by means of a sequence including:
a) an addition of compounds IV to compounds such as R¹⁰R¹¹C=0, which leads to compounds having formula VI, b) the conversion of compounds having formula VI to compounds having formula VII by i) conversion of the hydroxy group of VI into a good leaving group such as a halogen atom or an ester derived from a carboxylic acid or from a sulfonic acid by the reaction with acylation or sulfonylation agents, followed by ii) elimination of the leaving group, and c) cyclization of VII by means of an intramolecular Michael-type addition (scheme 3, example 6 + 7).

The conversion of compounds having formula IV into compounds having formula V can be carried out in practice by both isolating one or several of the intermediates obtained in each step (examples 6 + 7), and by carrying out all the steps successively, one after the other, in the same reactor without isolating the intermediate products (example 8).

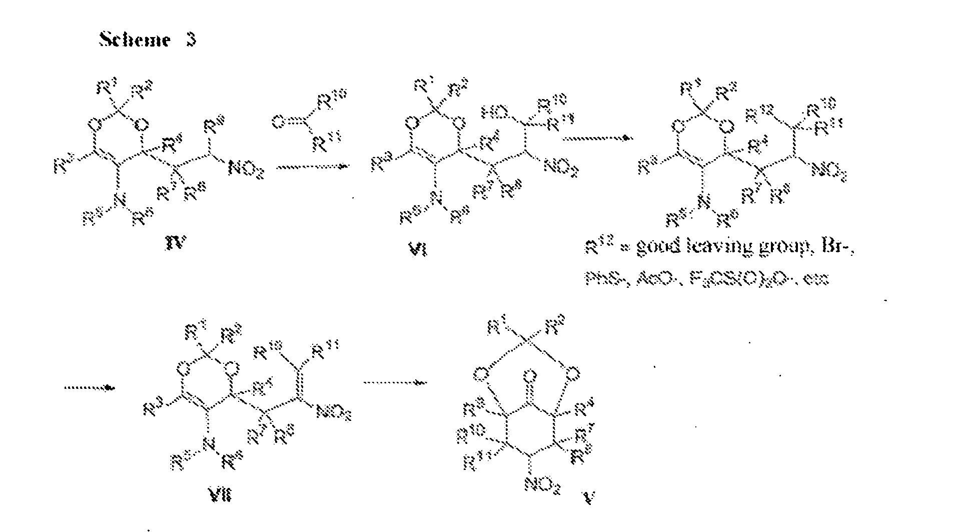

The novel and very convenient preparation of nitroolefins III having a group R⁹ = CHO by means of the oxidation of their corresponding precursor alcohols having formula VIII (Scheme 4, example 9) must also be emphasized.

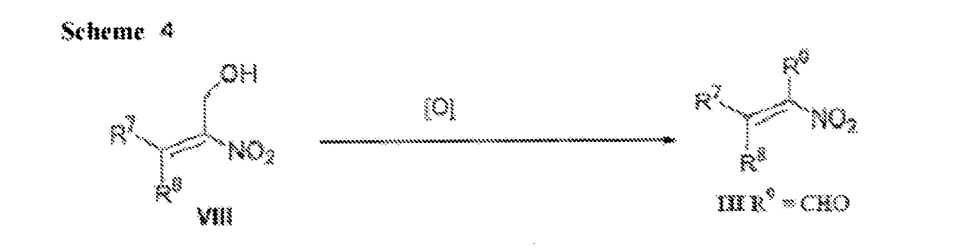

It is evident from their structural examination that both the compounds of type IV and those of type V are extraordinarily versatile precursors of other, very diverse polyoxygenated nitrogen systems into which they can be transformed by means of the suitable combination of simple reactions. In fact, several of these transformations of the systems IV and V have been successfully explored, guided by their synthetic use as precursors of the polyoxygenated nitrogen systems referred to in the introduction: certain antibiotics, tetrodotoxin and the analogues thereof, and pancratistatin and the analogues thereof. In practice, this confers an extraordinary added value to the synthetic methodology claimed herein as a route for accessing said systems IV and V and to the derivatives thereof by extension.

### Examples

The following examples are included below only for the purpose of contributing to a better understanding of the invention. The examples are by no means limited to the field of application of the described invention.

### Example 1. Preparation of enamine IIa starting from ketone Ia.

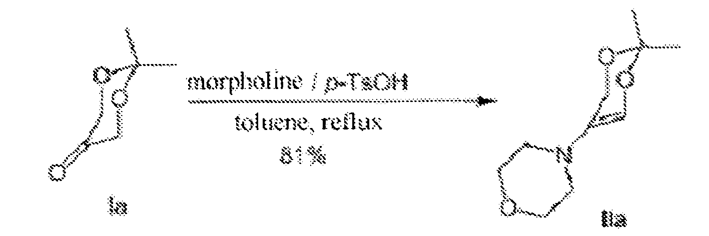

A solution of ketone Ia (4.12 g, 31.64 mmol), morpholine (3.3 mL, 37.97 mmol) and p-toluenesulfonic acid (307 mg) in toluene (250 mL, 1.9 M) was subjected to reflux for 5 h with azeotropic elimination of water by means of a Dean-Stark. The solvent was eliminated under reduced pressure and the raw product was redissolved in Et₂O, washed with a saturated NaHCO₃ solution, dried over anhydrous Na₂SO₄ and concentrated in a rotary evaporator. After a fast filtration through neutral alumina (activity I) (AcOEt-hexane 20:80), enamine IIa (5.08 g, 81%) was obtained as a colorless oil.
**¹H-NMR** (CDCl₃, 250 MHz, TMS) δ: 5.99 (t, *J*= 1.3 Hz, 1H, C*H*), 4.20 (d, *J*= 1.3 Hz, 2H, C*H*₂), 3.74-3.70 (m, 4H, 2xC*H*₂), 2.70-2.66 (m, 4H, 2xC*H*₂), 1.45 (s, 6H, 2xC*H*₃).
**¹³C-NMR** and **DEPT** (63 MHz) δ: 125.8 (C), 125.5 (*C*H), 97.9 (*C*), 66.7 (2x*C*H₂), 59.3 (*C*H₂), 50.0 (2x*C*H₂), 24.0 (2x*C*H₃).
**MS** (low resolution EI) m/z (%): 239 (M⁺, 0.03), 141 (2), 58 (100).
**MS** (low resolution CI⁺) m/z (%): 201 (M⁺+2, 16), 200 (M⁺+1, 80), 199 (M', 55), 170 (59), 143 (37), 142 (100), 141 (enal generated by the retro-hetero-Diels-Alder reaction of the dioxenone ring, 91), 112 (42), 59 (73).

### Example 2. Preparation of IVa starting from enamine IIa and nitroolefin IIIa.

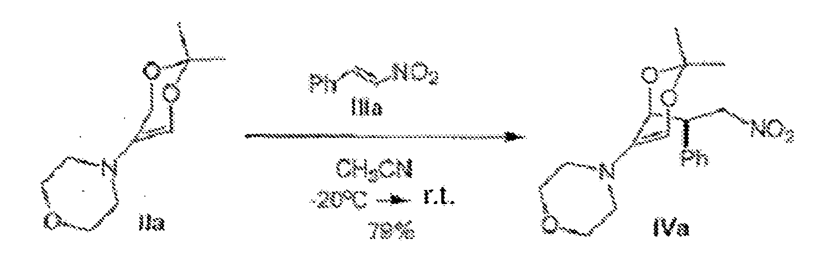

Nitroolefin IIIa (441 mg, 2.90 mmol) dissolved in acetonitrile was added to a solution of enamine IIa (583 mg, 2.90 mmol) in dry acetonitrile (1.5 mL) under argon at -20°C. The reaction was followed by means of thin layer chromatography (AcOEt-hexane 20:80) and after 12 h at room temperature (r.t.) the disappearance of the starting substance was observed. The reaction mixture was diluted with Et₂O and washed with water. The organic phase was dried with anhydrous Na₂SO₄ and the purification of the obtained residue by means of column chromatography (AcOEt-hexane, 10:90) led to polyoxygenated nitrogen system IVa (796 mg, 79%).
*Spectroscopic data of IVa in CDCl₃*:
**¹H-NMR** (CDCl₃, 250 MHz, TMS) δ: 7.39-7.35 (m, 2H, Ar*H*), 7.25-7.22 (m, 3H, Ar*H*), 5.76 (s, 1H, C*H*), 4.94 (dd, *J*= 13.5, *J*= 8.5, 1H, C*H*₂NO₂), 4.67 (dd, *J*= 13.5, *J*= 6.9, 1H, C*H*₂NO₂), 4.66 (d, *J*= 2.2, 1H, C*H*), 3.99 (ddd, *J*= 8.5, *J*= 6.9, *J*= 2.8, 1H, C*H*Ar), 3.72-3.68 (m, 4H, 2xC*H*₂), 2.75-2.67 (m, 2H, C*H*₂), 2.23-2.15 (m, 2H, C*H*₂), 1.47 (s, 3H, C*H*₃), 1.44 (s, 3H, C*H*₃).
**¹³C-NMR** and **DEPT** (63 MHz) δ: 135.8 (*Ar*), 129.6 (CH + *Ar*H), 127.7 (*Ar*H), 127.5 (*Ar*H), 126.6 (*C*), 98.1 (*C*), 76.8 (*C*H₂NO₂), 67.7 (*C*H), 66.8 (2x*C*H₂), 50.5 (2x*C*H₂), 45.1 (*C*HAr), 27.7 (*C*H₃), 20.3 (*C*H₃).
**MS** (low resolution EI) m/z (%): 348 (M⁺, 1), 290 (α,β-unsaturated ketone generated by the retro-hetero-Diels-Alder reaction of the dioxenone ring, 4), 244 (16), 230 (18), 156 (100), 126 (60), 115 (28), 91 (27), 77 (15).
*Spectroscopic data of IVa in CO(CD₃)₂*:
**¹H-NMR** (CO(CD₃)₂, 250 MHz, TMS) δ: 7.37-7.33 (m, 2H, Ar*H*), 7.16-7.07 (m, 3H, Ar*H*), 5.68 (s, 1H, CH), 4.92 (dd, *J*= 13.5, *J*= 6.6, 1H, C*H*₂NO₂), 4.81 (dd, *J*= 13.2, *J*= 4.1, 1H, C*H*₂NO₂), 4.73 (d, *J*= 2.8, 1H, C*H*), 3.95 (ddd, *J* 9.1, *J*= 6.6, *J*= 2.8, 1H, C*H*Ar), 3.58-3.55 (m, 4H, C*H*₂), 2.74-2.64 (m, 2H, C*H*₂), 2.10-2.02 (m, 2H, C*H*₂), 1.33 (s, 3H, C*H*₃), 1.30 (s, 3H, C*H*₃).
**¹³C-NMR** and **DEPT** (63 MHz) δ: 138.2 (*C*), 131.8 (*C*H), 130.9 (*C*H), 129.2 (*C*H), 128.9 (*C*H), 128.8 (*C*), 99.5 (*C*), 79.3 (*C*H₂NO₂), 70.2 (*C*H), 68.2 (2x*C*H₂), 52.4 (2x*C*H₂), 47.1 (*C*HAr), 29.0 (*C*H₃), 21.6 (*C*H₃).

### Example 3. Preparation of the bicyclic polyoxygenated nitrogen compound Va by the reaction of enamine IIb and nitroolefin IIIb.

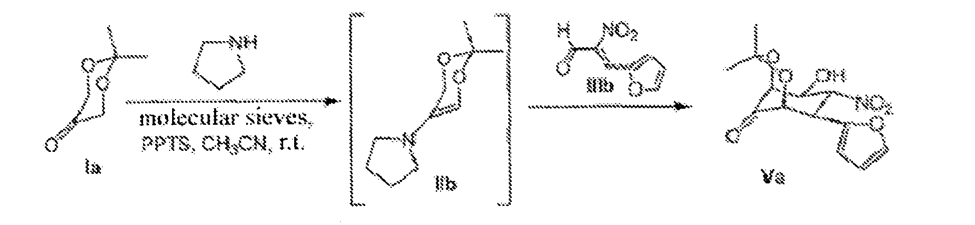

A solution of ketone Ia (50 mg, 0.38 mmol), pyrrolidine (39 µl, 0.46 mmol), molecular sieves (100 mg) and (catalytic) PPTS in dry acetonitrile (4 mL, 0.11 M) under argon was stirred for 40 min at r.t. Nitroolefin IIIb (71 mg, 0.42 mmol) was added to the reaction mixture at 0°C containing the enamine IIb thus prepared, and after 40 min at 0°C a mixture of 0.1 M HCl/acetone (5:20) was added. After 1 h at 0°C, the reaction mixture was washed with an NH₄Cl solution, and was extracted with Et₂O. The organic phase was dried over anhydrous Na₂SO₄, concentrated in a rotary evaporator and the raw product was purified by means of column chromatography (AcOEt-hexane 10:90), Va being obtained (51 mg, 45%).

### Example 4. Preparation of bicyclic polyoxygenated nitrogen compounds Va and Vb and of nitrogen systems IVb and IVc by the reaction of enamine IIa and nitroolefin IIIb.

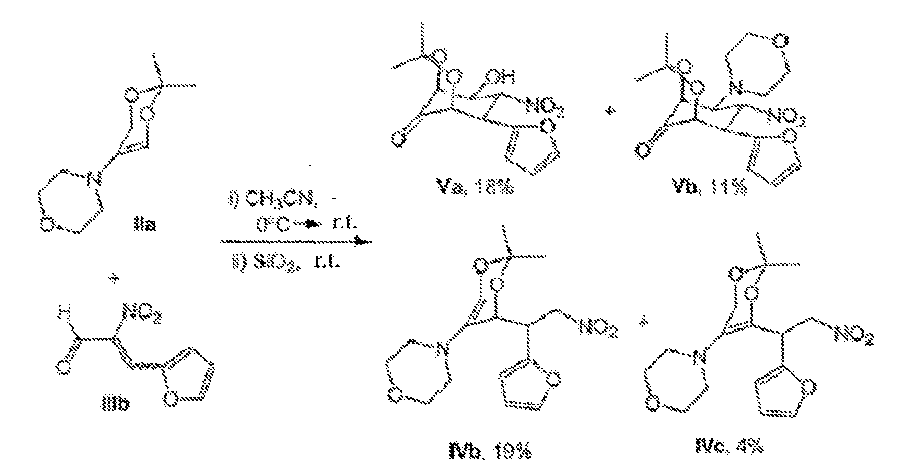

Nitroolefin IIIb (423 mg, 2.61 mmol) was added to a solution of enamine IIa (520 mg, 2.61 mmol) in acetonitrile (13 mL, 0.2 M) under argon at 0°C. After stirring for half an hour at 0°C and 4 h at r.t., SiO₂ was added and was left stirring for 2 days at r.t. After evaporating the solvent, column chromatography (AcOEt-hexane 10:90) led to bicycles Va (139 mg, 18%) and Vb (102 mg, 11%), to the trisubstituted enamine IVb (172 mg, 19%) and to its isomeric tetrasubstituted enamine IVc (39 mg, 4%).
*Spectroscopic data of bicycle Va:*
**¹H-NMR** (CDCl₃, 300 MHz, TMS) δ: 7.38 (d, *J*= 1.9, 1H, C*H*), 6.39 (d, *J*= 3.4, 1H, C*H*), 6.35 (dd, *J*= 3.4, *J*= 1.9, 1H, C*H*), 5.33 (dd, *J*= 11.6, *J*= 9.4, 1H, C*H*NO2), 4.55 (m, 1H, C*H*), 4.51 (m, 1H, C*H*), 4.20 (dd, *J*= 9.4, *J*= 1.3, 1H, C*H*OH), 3.51 (dd, *J*= 11.6, *J*= 1.3, 1H, C*H*), 3.01 (d, *J*=10.7, 1H, O*H*) , 1.57 (s, 3H, C*H*₃), 1.51 (s, 3H, C*H*₃).
**¹³C-NMR** and **DEPT** (63 MHz) δ: 205.0 (*C*O), 147.1 (*C*), 143.0 (*C*H), 110.7 (*C*H), 108.9 (*C*H), 99.9 (C), 89.0 (*C*HNO₂), 79.1 (*C*H), 77.0 (*C*H), 76.2 (*C*H), 44.4 (*C*H), 28.3 (*C*H₃), 25.5 (*C*H₃). **MS** (low resolution EI) m/z (%): 282 (17), 250 (24), 193 (57), 163 (50), 123 (100), 81 (80), 59 (69).
**IR** (KBr): 1739 (medium, narrow, CO), 1555 (intense, narrow, NO₂) cm⁻¹.
*Spectroscopic data of bicycle Vb:*
**¹H-NMR** (CDCl₃, 250 MHz, TMS) δ: 7.33 (s, 1H, C*H*), 6.36 (s, 1H, C*H*), 6.30 (s, 1H, C*H*), 5.60 (t, *J*= 11.0, 1H, C*H*NO₂), 4.77 (s, 1H, C*H*), 4.44 (s, 1H, C*H*), 3.67-3.49 (m, 5H, C*H* + 2xC*H*₂), 3.13 (d, *J*= 11.0, 1H, C*H*), 3.00-2.94 (m, 2H, C*H*₂), 2.60-2.53 (m, 2H, C*H*₂), 1.48 (s, 3H, C*H*₃), 1.42 (s, 3H, C*H*₃).
**¹³C-NMR** and **DEPT** (100 MHz) δ: 206.2 (*C*O), 147.8 *(C),* 142.8 (*C*H), 110.6 (*C*H), 108.7 (*C*H), 99.0 *(C),* 83.6 (*C*HNO₂), 77.3 (*C*H), 76.3 (*C*H), 72.5 (*C*H), 67.2 (2x*C*H₂), 50.2 (2x*C*H₂), 45.6 (*C*H), 28.2 (*C*H₃), 24.7 (*C*H₃).
**MS** (low resolution EI) m/z (%): 366 (M⁺, 2), 262 (12), 237 (4), 192 (100), 158 (42), 121 (84), 83 (41).
*Spectroscopic data of trisubstituted enamine IVb:*
**¹H-NMR** (CDCl₃, 250. MHz, TMS) δ: 7.28 (m, 1H, C*H*), 6.27 (m, 1H, C*H*), 6.21 (d, *J*= 3.6, 1H, C*H*), 5.90 (s, 1H, C*H*), 4.84-4.66 (m, 2H, CH₂NO₂), 4.64 (m, 1H, C*H*), 4.19 (*J*= 11.0, *J*= 7.3, *J*= 3.6, 1H, C*H*), 3.62-3.58 (m, 4H, 2xCH₂), 2.67-2.59 (m, 2H, C*H*₂), 2.35-2.26 (m, 2H, C*H*₂), 1.42 (s, 6H, 2xC*H*₃).
**¹³C-NMR** and **DEPT** (63 MHz) δ: 149.9 (*C*), 141.4 (*C*H), 129.7 (CH), 126.5 (*C*), 110.4 (CH), 107.7 (CH), 98.3 (*C*), 74.3 (*C*H₂NO₂), 67.0 (*C*H), 66.7 (2x*C*H₂), 50.6 (2x*C*H₂), 39.8 (*C*H), 27.7 (C*H*₃), 20.3 (*C*H₃).
**MS** (Low resolution EI) m/z (%): 338 (M⁺, 7), 280 (6), 234 (54), 220 (21), 156 (100), 128 (70), 94 (76), 65 (38).
*Spectroscopic data of tetrasubstituted enamine IVc:*
**¹H-NMR** (CDCl₃, 250 MHz, TMS) δ: 7.31 (s, 1H, C*H*), 6.29 (d, *J*= 3.6, 1H, C*H*), 6.14 (d, *J*= 3.6, 1H, C*H*), 5.49 (dd, *J*= 9.1, *J*= 5.5, 1H, C*H*), 4.81 (dd, *J*= 12.8, *J*= 9.1, 1H, C*H*₂NO₂), 4.67 (dd, *J*= 12.8, *J*= 5.5, 1H, C*H*₂NO₂), 4.24 (s, 2H, C*H*₂), 3.73-3.70 (m, 4H, 2xC*H*₂), 2.75-2.62 (m, 4H, 2xC*H*₂), 1.40 (s, 6H, 2xC*H*₃). **¹³C-NMR** and **DEPT** (75 MHz) δ: 150.5 (*C*), 141.7 (*C*H), 129.7 (*C*), 121.6 (*C*), 110.4 (*C*H), 106.7 (*C*H), 99.0 (*C*), 74.5 (*C*H₂NO₂), 67.4 (2x*C*H₂), 56.0 (*C*H₂), 51.4 (2x*C*H₂), 36.1 (*C*H), 24.7 (*C*H₃), 23.1 (*C*H₃).

### Example 5. Preparation of bicyclic polyoxygenated nitrogen compound Vc by the reaction of enamine IIb and nitroolefin IIIc.

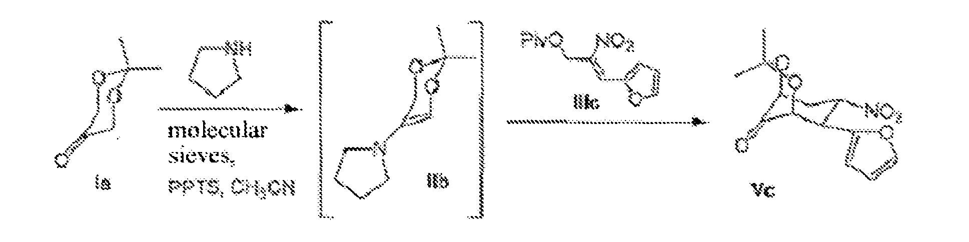

Pyrrolidine (21 µL, 0.25 mmol), molecular sieves (30 mg) and (catalytic) PPTS were added to a solution of ketone Ia (27 mg, 0.21 mmol) in acetonitrile (2 mL, 0.10 M), and the reaction mixture was stirred for 3.5 h at r.t. Nitroolefin IIIc (53 mg, 0.21 mmol) at 0°C was added, it was left stirring for one hour at this temperature and overnight at r.t. The reaction mixture was washed with a saturated NH₄Cl solution and was extracted with CH₂Cl₂. The organic phase was dried over anhydrous Na₂SO₄ and was concentrated in a rotary evaporator. The obtained residue was purified by means of column chromatography (CH₂Cl₂-hexane 40:60), the product Vc (19 mg, 31%) being obtained. Vc data:
**¹H-NMR** (CDCl₃, 500 MHz, TMS) δ: 7.36 (broad s, 1H, C*H*), 6.36 (d, *J*= 3.4, 1H, C*H*), 6.43 (dd, *J*= 3.4, *J*= 1.8, 1H, C*H*), 5.56 (ddd, *J*= 11.7, *J=* 11.4, *J*= 4.9, 1H, C*H*NO₂), 4.49 (t, *J*= 2.1, 1H, C*H*), 4.47-4.45 (m, 1H, C*H*), 3.81 (dd, *J*= 11.4, *J*=1.5, 1H, C*H*), 3.01 (dt, *J*= 13.2, *J*= 4.9, 1H, C*H*₂), 2.29 (ddd, *J*= 13.2, *J*= 11.7, *J*= 1.8, 1H, C*H*₂), 1.53 (s, 3H, C*H*₃), 1.45 (s, 3H, C*H*₃).
**¹³C-NMR** and **DEPT** (63 MHz) δ: 208.9 (*C*O), 149.0 (*C*), 142.7 (*C*H), 110.7 (*C*H), 108.3 (*C*H), 99.3 (*C*), 81.2 (*C*HNO₂), 77.3 (*C*H), 74.6 (*C*H), 49.9 (*C*H), 40.0 (*C*H₂), 28.4 (*C*H₃), 25.0 (*C*H₃).

### Example 6. Preparation of nitroolefin VIIa starting from IVa through VIa.

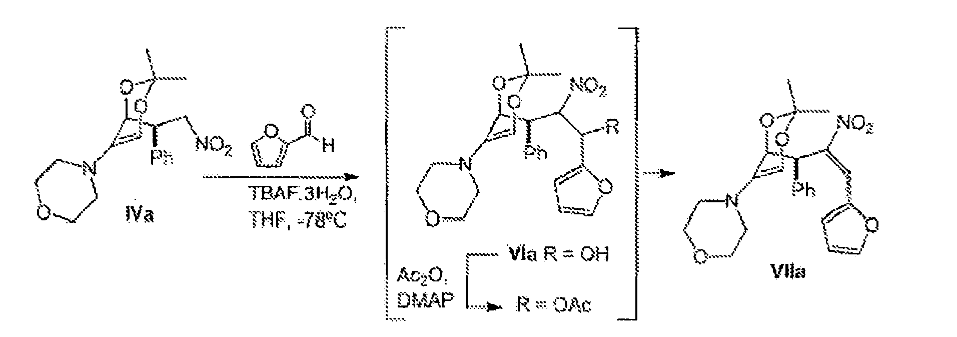

Furfural (171 µL, 2.10 mmol) and TBAF. 3H₂O (734 mg, 2.26 mmol) at -78°C were added to a solution of nitrocompound IVa (646 mg, 1.90 mmol) in dry THF (3.5 mL, 0.5 M) under argon. After 6 h of stirring, Ac₂O (218 µL, 2.26 mmol) and DMAP (69 mg, 0.56 mmol) were added. The mixture was stirred for 1.5 h at -78°C and 12 h at r.t. The resulting reaction mixture was diluted with Et₂O and was washed with a saturated NaHCO₃ solution. The organic phase was dried over anhydrous Na₂SO₄, the solvent was evaporated in a rotary evaporator and the resulting raw product was purified by means of column chromatography (AcOEt-hexane 10:90) to obtain nitroolefin VIIa as a yellow solid (88%).
*Spectroscopic data of VIIa:*
**¹H-NMR** (CDCl₃, 400 MHz, TMS) δ: 7.77 (s, 1H, C*H*), 7.65 (d, *J*= 1.9, 1H, C*H*), 7.40-7.37 (m, 2H, Ar*H*), 7.29-7.18 (m, 3H, Ar*H*), 6.90 (d, *J*= 3.4, 1H, *C*H), 6.59 (dd, *J*= 3.4, *J*= 1.9, 1H, C*H*), 6.24 (s, 1H, C*H*), 5.52 (d, *J* 8.7, 1H, C*H*), 5.23 (d, *J*= 8.7, 1H, C*H*Ar), 3.513.47 (m, 4H, C*H*₂), 2.85-2.77 (m, 2H, C*H*₂), 2.30-2.22 (m, 2H, C*H*₂), 1.51 (s, 3H, C*H*₃), 1.41 (s, 3H, C*H*₃).
**¹³C-NMR** and **DEPT** (100 MHz) δ: 150.0 (*C*), 147.8 (*C*), 146.1 (*C*H), 138.4 (*C*), 133.9 (*C*H), 131.0 (*C*), 128.3 (Ar*H*), 128.0 (ArH), 126.5 (ArH), 119.9 (*C*H), 119.3 (*C*H), 112.9 (*C*H), 99.2 (*C*), 67.4 (*C*H), 66.3 (2x*C*H₂), 52.3 (2x*C*H₂), 48.3 (*C*HAr), 28.0 (*C*H₃), 21.8 (*C*H₃).
**MS** (Low resolution EI) m/z (%): 368 (α,β-unsaturated ketone generated by the retro-hetero-Diels-Alder reaction of the dioxenone ring, 3), 182 (77), 153 (32), 128 (30), 58 (100).

### Example 7. Preparation of the bicyclic polyoxygenated nitrogen systems Vd and Ve.

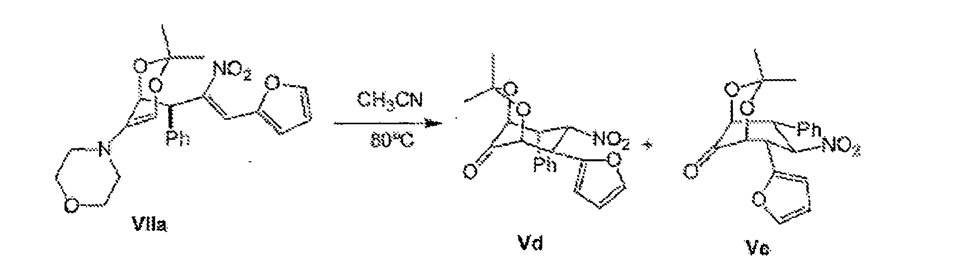

A solution of alkene VIIa (33 mg, 0.08 mmol) in acetonitrile (0.3 mL, 0.2 M) was heated at 80°C for 30 h. The reaction mixture was concentrated in the rotary evaporator and purified by means of column chromatography (AcOEt 10:90), obtaining the bicycles Vd (6 mg, 22%) and Ve (3 mg, 10%).
*Spectroscopic data of bicycle Ve:*
**¹H-NMR** (CDCl₃, 250 MHz, TMS) δ: 7.36 (dd, *J*= 1.9, *J*= 0.9, 1H, C*H*), 7.32-7.27 (m, 3H, Ar*H*), 7.18-7.14 (m, 2H, Ar*H*), 6.28 (dd, *J*= 3.1, *J*= 1.9, 1H, C*H*), 6.23-6.21 (d, *J*= 3.1, 1H, CH), 4.84 (dd, *J*= 10.7, *J*= 10.4, 1H, C*H*NO2), 4.61 (dd, *J*= 10.4, *J*= 2.5, C*H*Ar), 4.48 (d, *J*= 2.5, 1H, CH), 4.47 (dd, *J*= 10.7, *J*= 2.2, 1H, C*H*), 4.42-4.40 (m, 1H, C*H*), 1.62 (s, 3H, C*H*3), 1.46 (s, 3H, C*H*3).
**¹³C-NMR** and **DEPT** (63 MHz) δ: 207.8 (*C*O), 148.8 (*C*), 143.3 (*C*H), 136.8 (Ar), 129.2 (ArH), 128.4 (ArH), 127.5 (ArH), 110.5 (CH), 108.7 (CH), 101.9 (C), 87.5 (*C*HNO2), 78.6 (CH), 76.3 (CH), 54.6 (*C*HAr), 48.5 (CH), 29.7 (*C*H3), 25.9 (*C*H3).
**MS** (low resolution CI⁺) m/z (%): 358 (M⁺+1, 2), 252 (42), 223 (100).
**IR** (CsI): 1759 (intense, narrow, CO), 1561 (intense, narrow, NO₂) cm⁻¹.

### Example 8. Preparation of bicycles Vd and Ve starting from IVa.

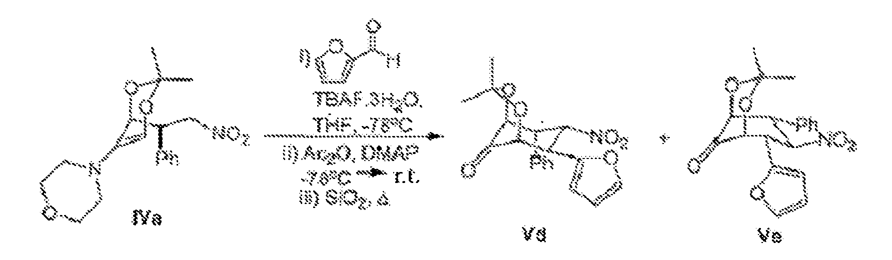

Furfural (12 µL, 0.14 mmol) and TBAF. 3H₂O (54 mg, 0.16 mmol) at -78°C were added to a solution of nitrocompound IVa (44 mg, 0.13 mmol) in dry CH₂Cl₂ (0.3 mL, 0.4 M) under argon. After 7 h of stirring, Ac₂O (15 µL, 0.15 mmol) and DMAP (5 mg, 0.04 mmol) were added. The mixture was stirred for 2.5 h at - 78°C and 15 h at r.t. Then, SiO₂ (≅ 44 mg) was added to the reaction mixture and after 6 h under reflux, the solvent was evaporated under vacuum. Bicycles Vd (6 mg, 14%), and Ve (2 mg, 4%) were obtained after purifying the raw product by means of column chromatography (AcOEt-hexane 10:90). The starting nitrocompound IVa was partially recovered (13 mg, 30%).

### Example 9. Preparation of nitroolefin IIIb.

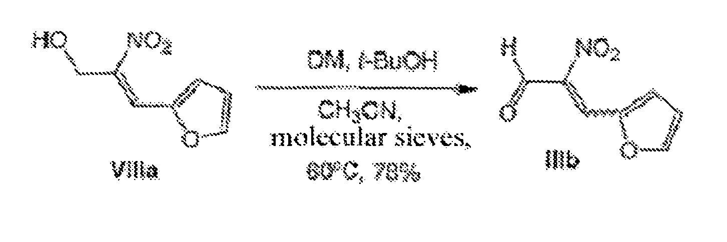

Dess Martin reagent (1.69 g, 3.98 mmol), *t*-butanol (562 µL, 5.97 mmol) and molecular sieves were added to a solution of alcohol VIIIa (672 mg, 3.98 mmol) in dry acetonitrile (25 mL, 0.16 M) under argon at 60°C. After half an hour, the reaction mixture was diluted with Et₂O, it was filtered and the solvent was evaporated under reduced pressure. The raw product was purified by means of a fast filtration through silica gel (AcOEt-hexane 20:80), nitroolefin IIIb being obtained as an E/Z mixture (3:2) (1.58 g, 78%).
*Spectroscopic data of the E*/*Z mixture IIIb:*
**¹H-NMR** (CDCl₃, 500 MHz, TMS) δ: 10.38 (dd, *J*= 2.3, 1H, C*H*-E), 9.81 (s, 1H, C*H*-Z), 8.45 (d, *J*= 3.7, 1H, C*H*-E), 8.36 (d, *J*= 2.3, 1H, C*H*-E), 7.92 (d, *J*= 1.6, 1H, C*H*-E), 7.82 (d, *J*= 1.6, 1H, C*H*-Z), 7.70 (d, *J*= 3.7, 1H, C*H*-Z), 7.52 (s, 1H, C*H*-Z), 6.77 (dd, *J*= 3.7, *J*= 1.6, 1H, C*H*-E), 6.74 (dd, *J*= 3.7, *J*= 1.6, 1H, CH-Z).
**¹³C-NMR** and **DEPT** (63 MHz) δ: 183.6 (*C*HO), 182.1 (*C*HO), 152.0 (*C*H), 150.6 (*C*H), 147.4 (*C*), 146.6 (*C*), 130.0 (*C*H), 128.3 (*C*H), 126.3 (*C*H), 125.3 (*C*H), 115.4 (*C*H), 114.9 5 (*C*H).
**MS** (low resolution EI) m/z (%): 167 (M⁺, 100), 121 (M⁺-NO₂, 2), 106 (21), 83 (24), 63 (22), 58 (100).

## Claims

1. Compounds having formula IV 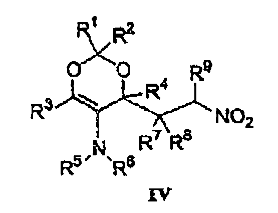 wherein R⁹ is a hydrogen or a halogen atom or a linear, branched or cyclic alkyl group, an alkenyl, aryl, heterocyclic, alkyloxy, acyloxy, aryloxy, thioalkyloxy, amino, alkylamino, arylamino, hydroxycarbonyl, alkoxycarbonyl, aryloxycarbonyl, thiocarbonyl, aminocarbonyl or hydroxy group, said groups being optionally substituted by one or several identical or different substituents selected from halogen atoms, alkyl, aryl, hydroxy, alkyloxy, acyloxy, aryloxy, amino, alkylamino, arylamino, heterocyclic, hydroxycarbonyl, alkoxycarbonyl, aryloxycarbonyl or aminocarbonyl groups, wherein R⁷ and R⁸ can have the same values as R⁹ except alkenyl, wherein R⁵ and R⁶ can be alkinyl and have the same values as R⁹ except acyloxy, hydroxycarbonyl, alkoxycarbonyl, thiocarbonyl and aminocarbonyl, wherein R³ and R⁴ can be alkinyl and have the same values as R⁹ except acyloxy, aryloxy, alkyloxy, thioalkyloxy, amino, alkylamino, arylamino and hydroxy, wherein R¹ and R² can be alkinyl and have the same values as R⁹ except amino, alkylamino, arylamino and hydroxy, wherein R¹ and R² can further jointly be a carbonyl or thiocarbonyl group.

2. Compounds having formula V 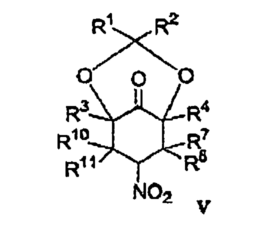 wherein the R¹, R², R³, R⁴, R⁷ and R⁶ groups can have the same values as those indicated for said groups in claim 1, wherein R¹⁰ and R¹¹ can be Alkinyl, hydrogen or a halogen atom or a linear, branched or cyclic alkyl group, an alkenyl, aryl, heterocyclic, amino, alkylamino, arylamino, or hydroxy group, said groups being optionally substituted by one or several identical or different substituents selected from halogen atoms, alkyl, aryl, hydroxy, alkyloxy, acyloxy, aryloxy, amino, alkylamino, arylamino, heterocyclic, hydroxycarbonyl, alkoxycarbonyl, aryloxycarbonyl or aminocarbonyl groups, wherein R¹⁰ and R¹¹ can further jointly be a carbonyl group.

3. A process for preparing compounds having formula IV **characterized by** the reaction of an enamine II with a nitroolefin III, wherein the R¹, R², R³, R⁴, R⁵, R⁶, R⁷, R⁸ and R⁹ groups can have the same values as those indicated for said groups in claim 1.

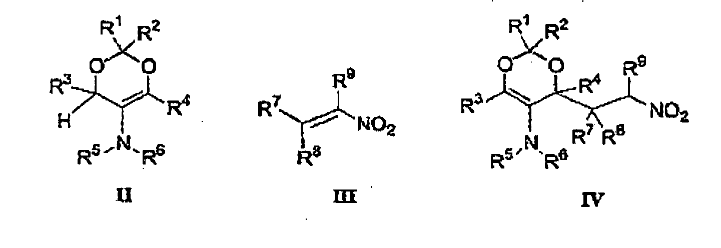

4. A process for preparing compounds having formula V' **characterized by** the reaction of an enamine II with a nitroolefin III wherein R⁹ = C(O)X wherein X can be a hydrogen or a halogen atom or O, S, Se or Te atoms joined to an alkyl, aryl or acyl group, wherein the R¹, R², R³, R⁴, R⁵, R⁶, R⁷ and R⁸ groups can have the same values as those indicated for said groups in claims 1 and. 2 and wherein R^{10'} is hydrogen and R^{11'} can be hydroxyl, optionally substituted amino by one or several identical or different substituents selected from halogen atoms, alkyl, aryl, hydroxy, alkyloxy, acyloxy, aryloxy, amino, alkylamino, arylamino, heterocyclic, hydroxycarbonyl, alkoxycarbonyl, aryloxycarbonyl or aminocarbonyl groups; alkylamino, arylamino, and jointly R^{10'} and R^{11'} be a carbonyl group.

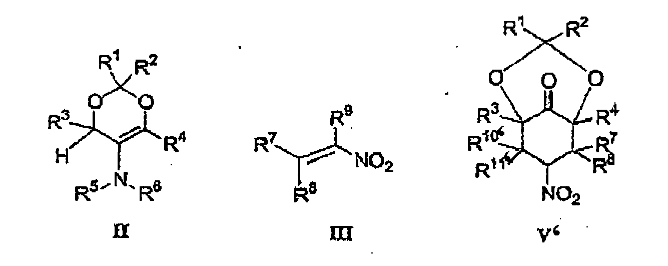

5. A process for preparing compounds having formula #V according to claim 2, **characterized by** the reaction of an enamine II with a nitroolefin III wherein R⁹ = CXYZ, wherein X and Y can be hydrogen or halogen atoms or alkyl or aryl groups which can in turn be substituted by one or several identical or different substituents selected from halogen atoms, alkyl, aryl, hydroxy, alkoxy, aryloxy, amino., alkylamino or arylamino groups, and Z is any leaving group such as: a) a halogen atom, or b) an -OC(O)R^{b} group, or c) an -OS(O)ₙR^{b} wherein n can have the values 1 or 2, and R^{b} can be a linear, branched or cyclic alkyl group or an aryl group, which groups can in turn be substituted by one or several identical or different substituents selected from halogen atoms, alkyl, hydroxy, alkoxy, aryloxy, amino or alkylamino groups and wherein the R¹, R², R³, R⁴, R⁵, R⁶, R⁷, and R⁸ groups can have the same values as those indicated for said groups in claims 1 and 2; and #R¹⁰ and #R¹¹ can be hydrogen or halogen atoms or alkyl or aryl groups which can in turn be substituted by one or several identical or different substituents selected from halogen atoms, alkyl, aryl, hydroxy, alkoxy, aryloxy, amino, alkylamino or arylamino groups.

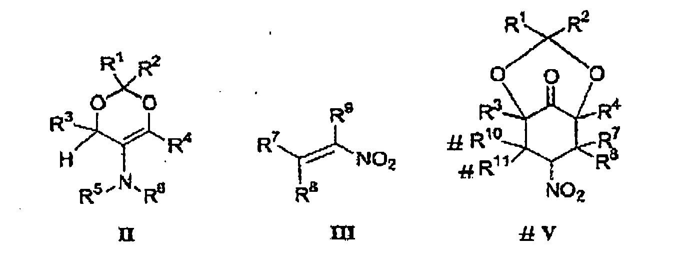

6. A process for preparing compounds having formula VI **characterized by** an addition reaction of a compound having formula IV to a carbonyl compound *R¹⁰*R¹¹(C=O), wherein the R¹, R², R³, R⁴, R⁵, R⁶, R⁷, R⁸, R⁹ groups can have the same values as those indicated for said groups in claims 1 and 2; and *R¹⁰ and *R¹¹ are hydrogen, a linear, branched or cyclic alkyl group, an alkenyl, an alkynyl, aryl, heterocyclic, amino, alkylamino, or arylamino, said groups being optionally substituted by one or several identical or different substituents selected from halogen atoms, alkyl, aryl, hydroxy, alkyloxy, acyloxy, aryloxy, amino, alkylamino, arylamino, heterocyclic, hydroxycarbonyl, alkoxycarbonyl, aryloxycarbonyl or aminocarbonyl groups.

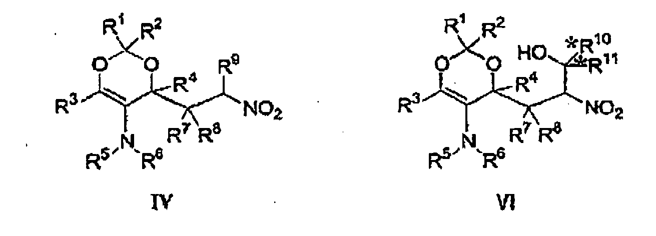

7. A process for preparing compounds having formula VII **characterized by** a) a conversion reaction of the hydroxy group of a compound having formula VI into a good leaving group such as a halogen atom, an SR, -OC(O)R or -OS(O)ₙR group, wherein n can have the values 1 and 2 and R can be a linear, branched or cyclic alkyl group or an aryl group, which groups can in turn be substituted by one or several identical or different substituents selected from halogen atoms, alkyl, hydroxy, alkoxy, aryloxy, amino, alkylamino or arylamino groups followed by b) the elimination of said leaving group and wherein the R¹, R², R³, R⁴, R⁵, R⁶, R⁷, R⁸, *R¹⁰ and *R¹¹ groups can have the same values as those indicated for said groups in claim 6.

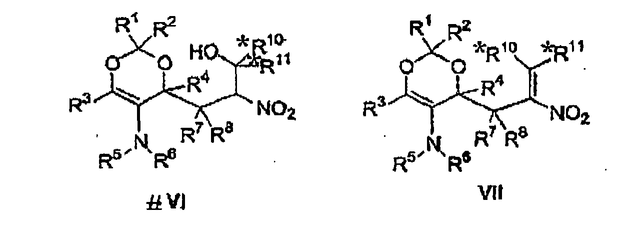

8. A process for preparing compounds having formula *V **characterized by** the cyclization of a compound having formula VII according to claim 7, by means of an intramolecular Michael-type addition, wherein the R¹, R², R³, R⁴, R⁵, R⁶, R⁷, R⁸, *R¹⁰ and *R¹¹ groups can have the same values as those indicated for said groups in claim 6.

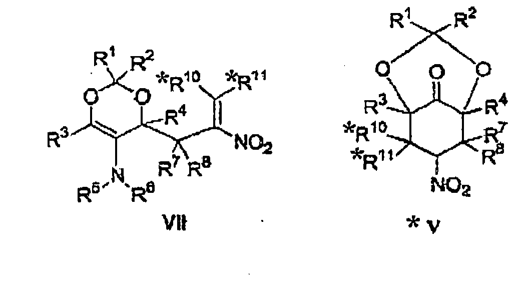

## Patentansprüche

1. Verbindungen mit der Formel IV 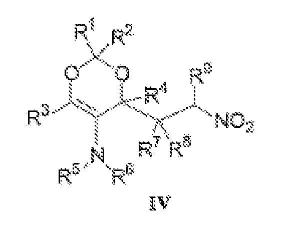 wobei R⁹ ein Wasserstoff- oder Halogenatom oder eine lineare, verzweigte oder cyclische Alkylgruppe, eine Alkenyl-, Aryl-, heterocyclische, Alkyloxy-, Acyloxy-, Aryloxy-, Thioalkyloxy-, Amino-, Alkylamino-, Arylamino-, Hydroxycarbonyl-, Alkoxycarbonyl-, Aryloxycarbonyl-, Thiocarbonyl-, Aminocarbonyl- oder Hydroxy-Gruppe ist, wobei die Gruppen optional substituiert sind mit einem oder mehreren identischen oder unterschiedlichen Substituenten ausgewählt aus Halogenatomen, Alkyl-, Aryl-, Hydroxy-, Alkyloxy-, Acyloxy-, Aryloxy-, Amino-, Alkylamino-, Arylamino-, heterocyclische, Hydroxycarbonyl-, Alkoxycarbonyl-, Aryloxycarbonyl- oder Aminocarbonyl-Gruppen, wobei R⁷ und R⁸ die gleichen Werte wie R⁹ haben können, außer Alkenyl, wobei R⁵ und R⁶ Alkinyl sein können und die gleichen Werte wie R⁹ haben, außer Acyloxy, Hydroxycarbonyl, Alkoxycarbonyl, Thiocarbonyl und Aminocarbonyl, wobei R³ und R⁴ Alkinyl sein können und die gleichen Werte wie R⁹ haben, außer Acyloxy, Aryloxy, Alkyloxy, Thioalkyloxy, Amino, Alkylamino, Arylamino und Hydroxy, wobei R¹ und R² Alkinyl sein können und die gleichen Werte wie R⁹ haben, außer Amino, Alkylamino, Arylamino und Hydroxy, wobei R¹ und R² ferner gemeinsam eine Carbonyl- oder Thiocarbonylgruppe sein können.

2. Verbindungen mit der Formel V 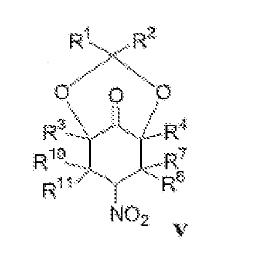 wobei die R¹-, R²-, R³-, R⁴-, R⁷- und R⁸-Gruppen die gleichen Werte, wie die für die Gruppen in Anspruch 1 angezeigten, haben können, wobei R¹⁰ und R¹¹ Alkinyl, Wasserstoff oder ein Halogenatom oder eine lineare, verzweigte oder cyclische Alkylgruppe, eine Alkenyl-, Aryl-, heterocyclische, Amino-, Alkylamino-, Arylamino- oder Hydroxygruppe sein können, wobei die Gruppen optional substituiert sind mit einem oder mehreren identischen oder unterschiedlichen Substituenten ausgewählt aus Halogenatomen, Alkyl-, Aryl-, Hydroxy-, Alkyloxy-, Acyloxy-, Aryloxy-, Amino-, Alkylamino-, Arylamino-, heterocyclische, Hydroxycarbonyl-, Alkoxycarbonyl-, Aryloxycarbonyl- oder Aminocarbonyl-Gruppen, wobei R¹⁰ und R¹¹ ferner gemeinsam eine Carbonylgruppe sein können.

3. Verfahren zur Herstellung von Verbindungen mit der Formel IV, **gekennzeichnet durch** die Reaktion eines Enamins II mit einem Nitroolefin III, wobei die R¹-, R²-, R³-, R⁴-, R⁵-, R⁶-, R⁷-, R⁸- und R⁹-Gruppen die gleichen Werte, wie die für die Gruppen in Anspruch 1 angezeigten, haben können.

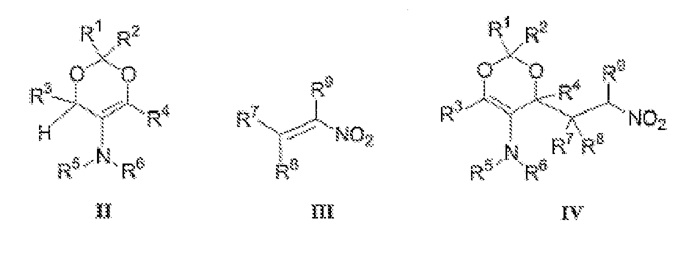

4. Verfahren zur Herstellung von Verbindungen mit der Formel V', **gekennzeichnet durch** die Reaktion eines Enamins II mit einem Nitroolefin III, wobei R⁹ = C(O)X, wobei X ein Wasserstoff- oder ein Halogenatom oder O-, S-, Se- oder Te-Atome gebunden an eine Alkyl-, Aryl- oder Acylgruppe sein kann, wobei die R¹-, R²-, R³-, R⁴-, R⁵-, R⁶-, R⁷- und R⁸-Gruppen die gleichen Werte, wie die für die Gruppen in den Ansprüchen 1 und 2 angezeigten, haben können und wobei R^{10'} Wasserstoff ist und R^{11'} Hydroxyl sein kann, optional substituiertes Amino mit einem oder mehreren identischen oder unterschiedlichen Substituenten ausgewählt aus Halogenatomen, Alkyl-, Aryl-, Hydroxy-, Alkyloxy-, Acyloxy-, Aryloxy-, Amino-, Alkylamino-, Arylamino-, heterocyclische, Hydroxycarbonyl-, Alkoxycarbonyl-, Aryloxycarbonyl- oder Aminocarbonyl-Gruppen; Alkylamino, Arylamino, und R^{10'} und R^{11'} gemeinsam eine Carbonylgruppe sind.

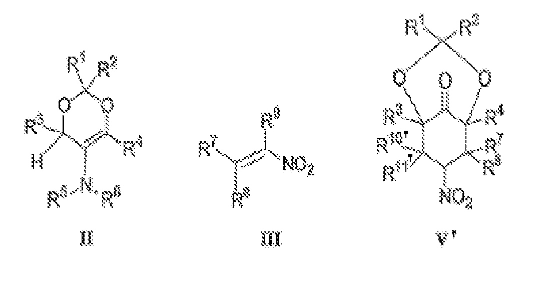

5. Verfahren zur Herstellung von Verbindungen mit der Formel #V gemäß Anspruch 2, **gekennzeichnet durch** die Reaktion eines Enamins II mit einem Nitroolefin III, wobei R⁹ = CXYZ, wobei X und Y Wasserstoff- oder Halogenatome oder Alkyl- oder Arylgruppen sein können, welche wiederum substituiert sein können mit einem oder mehreren identischen oder unterschiedlichen Substituenten ausgewählt aus Halogenatomen, Alkyl-, Aryl-, Hydroxy-, Alkoxy-, Aryloxy-, Amino-, Alkylamino- oder Arylaminogruppen, und Z ist eine Abgangsgruppe wie etwa: a) ein Halogenatom, oder b) eine -OC(O)R^{b} Gruppe, oder c) ein -OS(O)ₙR^{b}, wobei n die Werte 1 oder 2 aufweisen kann, und R^{b} eine lineare, verzweigte oder cyclische Alkylgruppe oder eine Arylgruppe sein kann, welche Gruppen wiederum substituiert sein können mit einem oder mehreren identischen oder unterschiedlichen Substituenten ausgewählt aus Halogenatomen, Alkyl-, Hydroxy-, Alkoxy-, Aryloxy-, Amino- oder Alkylaminogruppen und wobei die R¹-, R²-, R³-, R⁴-, R⁵-, R⁶-, R⁷- und R⁸-Gruppen die gleichen Werte, wie die für die Gruppen in Anspruch 1 und 2 angezeigten, haben können; und #R¹⁰ und #R¹¹ können Wasserstoff- oder Halogenatome oder Alkyl- oder Arylgruppen sein, welche wiederum substituiert sein können mit einem oder mehreren identischen oder unterschiedlichen Substituenten ausgewählt aus Halogenatomen, Alkyl-, Aryl-, Hydroxy-, Alkoxy-, Aryloxy-, Amino-, Alkylamino- oder Arylaminogruppen.

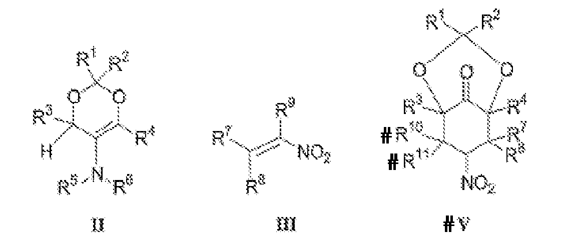

6. Verfahren zur Herstellung von Verbindungen mit der Formel VI, **gekennzeichnet durch** eine Additionsreaktion einer Verbindung mit der Formel IV an eine Carbonylverbindung *R¹⁰*R¹¹ (C=O), wobei die R¹-, R²-, R³-, R⁴-, R⁵-, R⁶-, R⁷-, R⁸-, R⁹-Gruppen die gleichen Werte, wie die für die Gruppen in Anspruch 1 und 2 angezeigten, haben können; und *R¹⁰ und *R¹¹ sind Wasserstoff, eine lineare, verzweigte oder cyclische Alkylgruppe, ein Alkenyl, ein Alkynyl, Aryl, Heterocyclisches, Amino, Alkylamino oder Arylamino, wobei die Gruppen optional substituiert sind mit einem oder mehreren identischen oder unterschiedlichen Substituenten ausgewählt aus Halogenatomen, Alkyl-, Aryl-, Hydroxy-, Alkyloxy-, Acyloxy-, Aryloxy-, Amino-, Alkylamino-, Arylamino-, heterocyclische, Hydroxycarbonyl-, Alkoxycarbonyl-, Aryloxycarbonyl- oder Aminocarbonyl-Gruppen.

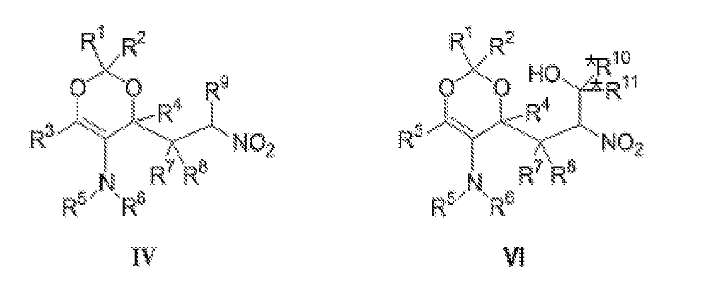

7. Verfahren zur Herstellung von Verbindungen mit der Formel VII, **gekennzeichnet durch** a) eine Konversionsreaktion der Hydroxygruppe einer Verbindung mit der Formel #VI in eine gute Abgangsgruppe wie etwa ein Halogenatom, eine SR, -OC(O)R oder -OS(O)ₙR Gruppe, wobei n die Werte 1 und 2 aufweisen kann und R eine lineare, verzweigte oder cyclische Alkylgruppe oder eine Arylgruppe sein kann, welche Gruppen wiederum substituiert sein können mit einem oder mehreren identischen oder unterschiedlichen Substituenten ausgewählt aus Halogenatomen, Alkyl-, Hydroxy-, Alkoxy-, Aryloxy-, Amino-, Alkylamino- oder Arylaminogruppen, gefolgt von b) der Eliminierung der Abgangsgruppe, und wobei die R¹-, R²-, R³-, R⁴-, R⁵-, R⁶-, R⁷-, R⁸-, *R¹⁰- und *R¹¹-Gruppen die gleichen Werte, wie die für die Gruppen in Anspruch 6 angezeigten, haben können.

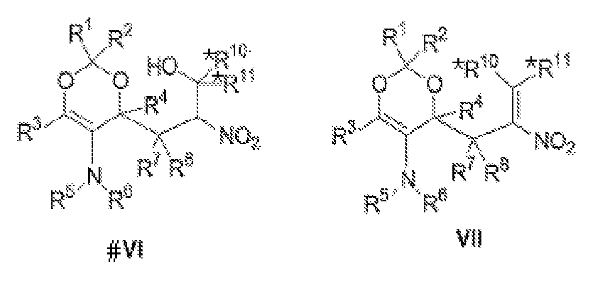

8. Verfahren zur Herstellung von Verbindungen mit der Formel *V, **gekennzeichnet durch** die Cyclisierung einer Verbindung mit der Formel VII gemäß Anspruch 7, mittels einer intramolekularen Michael-artigen Addition, wobei die R¹-, R²-, R³-, R⁴-, R⁵-, R⁶-, R⁷-, R⁸-, *R¹⁰- und *R¹¹-Gruppen die gleichen Werte, wie die für die Gruppen in Anspruch 6 angezeigten, haben können.

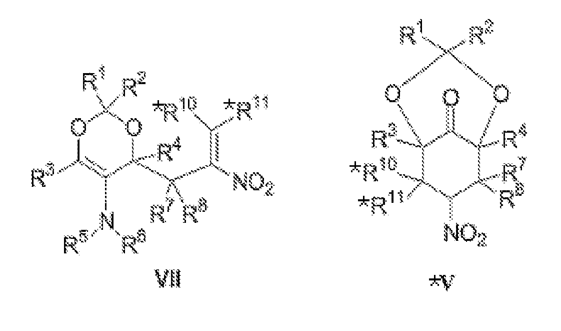

## Revendications

1. Composés ayant la formule IV 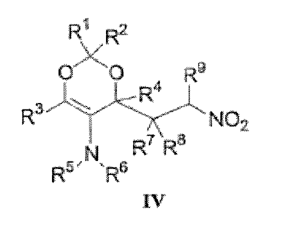 **caractérisés en ce que** R⁹ est un atome d'hydrogène ou d'un halogène ou un groupement alkyle linéaire, ramifié ou cyclique, un groupement alkényle, aryle, hétérocyclique, alkyloxy, acyloxy, aryloxy, thioalkyloxy, amino, alkylamino, arylamino, hydroxycarbonyle, alkoxycarbonyle, aryloxycarbonyle, thiocarbonyle, aminocarbonyle ou hydroxy, les dits groupements étant éventuellement remplacés par un ou plusieurs substituts identiques ou différents choisis parmi les atomes d'halogène, les groupements alkyle, aryle, hydroxy, alkyloxy, acyloxy, aryloxy, amino, alkylamino, arylamino, hétérocyclique, hydroxycarbonyle, alkoxycarbonyle, aryloxycarbonyle ou aminocarbonyle, où R⁷ et R⁸ peuvent avoir les mêmes valeurs que R⁹ sauf l'alkenyle, où R⁵ et R⁶ peuvent être de l'alkinyle et avoir les mêmes valeurs que R⁹ sauf l'acyloxy, l'hydroxycarbonyle, l'alkoxycarbonyle, le thiocarbonyle et l'aminocarbonyle, où R³ et R⁴ peuvent être de l'alkinyl et avoir les mêmes valeurs que R⁹ sauf l'acyloxy, l'aryloxy, l'alkyloxy, le thioalkyloxy, l'amino, l'alkylamino, l'arylamino et l'hydroxy, où R¹ et R² peuvent être de l'alkinyle et avoir les mêmes valeurs que R⁹ sauf l'amino, l'alkylamino, l'arylamino et l'hydroxy, où R¹ et R² peuvent de plus être conjointement un groupement carbonyle ou thiocarbonyle.

2. Composés ayant la formule V 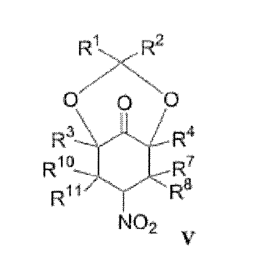 où les groupements R¹, R², R³, R⁴, R⁷ et R⁸ peuvent avoir les mêmes valeurs que celles indiquées pour les dits groupes dans la revendication 1, **caractérisés en ce que** R¹⁰ et R¹¹ peuvent être un alkinyle, un atome d'hydrogène ou d'halogène ou un groupement alkyle linéaire, ramifié ou cyclique, un groupement alkenyle, aryle, hétérocyclique, amino, alkylamino, arylamino ou hydroxy, les dits groupements étant éventuellement remplacés par un ou plusieurs substituts identiques ou différents choisis parmi les atomes d'halogène, les groupements alkyle, aryle, hydroxy, alkyloxy, acyloxy, aryloxy, amino, alkylamino, arylamino, hétérocyclique, hydroxycarbonyle, alkoxycarbonyle, aryloxycarbonyle ou aminocarbonyle, où R¹⁰ et R¹¹ peuvent de plus être conjointement un groupement carbonyle.

3. Procédé de préparation de composés ayant la formule IV **caractérisé par** la réaction d'une énamine II avec une nitrooléfine III, où les groupements R¹, R², R³, R⁴, R⁵, R⁶, R⁷, R⁸ et R⁹ peuvent avoir les mêmes valeurs que celles indiquées pour les dits groupements dans la revendication 1.

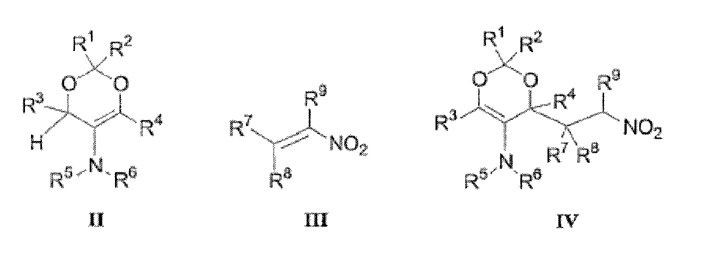

4. Procédé de préparation de composés ayant la formule V' **caractérisé par** la réaction d'une énamine II avec une nitrooléfine III dans laquelle R⁹=C(O)X où X peut être un atome d'hydrogène ou d'un halogène ou des atomes O, S, Se ou Te unis à un groupement alkyle, aryle ou acyle, où les groupements R¹, R², R³, R⁴, R⁵, R⁶, R⁷ et R⁸ peuvent avoir les mêmes valeurs que celles indiquées pour les dits groupes dans les revendications 1 et 2 et où R¹⁰ est un hydrogène et R¹¹ peut être un hydroxyl, un amino éventuellement remplacé par un ou plusieurs substituts identiques ou différents choisis parmi les atomes d'halogène, les groupements alkyle, aryle, hydroxy, alkyloxy, acyloxy, aryloxy, amino, alkylamino, arylamino, hétérocyclique, hydroxycarbonyle, alkoxycarbonyle, aryloxycarbonyle ou aminocarbonyle, alkylamino, arylamino, et R¹⁰ et R¹¹ peuvent être conjointement un groupement carbonyle.

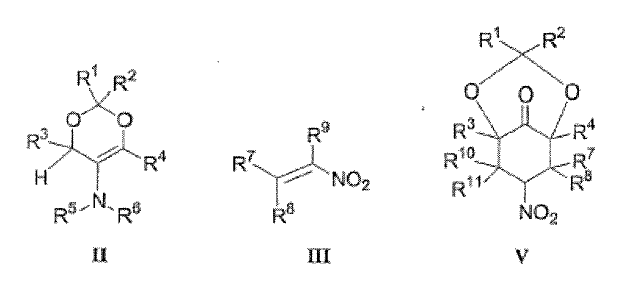

5. Procédé pour préparer des composés ayant la formule #V selon la revendication 2, **caractérisé par** la réaction d'une énamine II avec une nitrooléfine III où R⁹=CXYZ, où X et Y peuvent être des atomes d'hydrogène ou d'halogène ou des groupements alkyle ou aryle qui peuvent à leur tour être remplacés par un ou plusieurs substituts identiques ou différents choisis parmi les atomes d'halogène, des groupements alkyle, aryle, hydroxy, alkoxy, aryloxy, amino, alkylamino ou arylamino, et Z est n'importe quel groupe tel que : a) un atome d'halogène ou b) un groupement -OC(O)R^{b}, ou c) un -OS(O)ₙR^{b} où n peut avoir comme valeur 1 ou 2, et R^{b} peut être un groupement alkyle linéaire, ramifié ou cyclique ou un groupement aryle, ces groupements pouvant à leur tour être remplacés par un ou plusieurs substituts identiques ou différents choisis parmi les atomes d'halogène, les groupements alkyle, hydroxy, alkoxy, aryloxy, amino ou alkylamino et où les groupements R¹, R², R³, R⁴, R⁵, R⁶, R⁷ et R⁸ peuvent avoir les mêmes valeurs que celles indiquées pour les dits groupements dans les revendications 1 et 2, et #R¹⁰ et #R¹¹ peuvent être des atomes d'hydrogène ou d'halogène ou des groupements alkyle ou aryle qui peuvent à leur tour être remplacés par un ou plusieurs substituts identiques ou différents choisis parmi les atomes d'halogène, les groupements alkyle, aryle, hydroxy, alkoxy, aryloxy, amino, alkylamino ou arylamino.

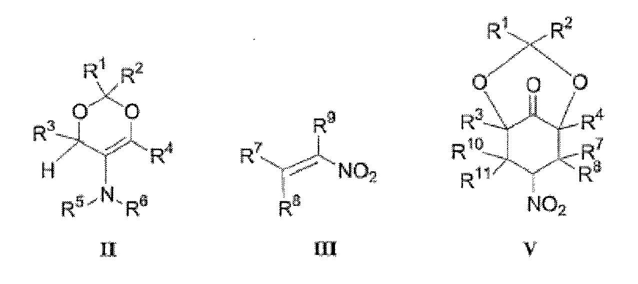

6. Procédé de préparation de composés ayant la formule VI **caractérisé par** une réaction d'addition d'un composé ayant la formule IV à un composé carbonyle *R¹⁰*R¹¹(C=O), où les groupements R¹, R², R³, R⁴, R⁵, R⁶, R⁷, R⁸, R⁹ peuvent avoir les mêmes valeurs que celles indiquées pour les dits groupes dans les revendications 1 et 2, et *R¹⁰ et *R¹¹ sont de l'hydrogène, ou un groupement alkyle linéaire, ramifié ou cyclique, un alkényle, un alkynyle, aryle, hétérocyclique, amino, alkylamino ou arylamino, les dits groupes étant éventuellement remplacés par un ou plusieurs substituts identiques ou différents choisis parmi les atomes d'halogène, les groupements alkyle, aryle, hydroxy, alkyloxy, acyloxy, aryloxy, amino, alkylamino, arylamino, hétérocyclique, hydroxycarbonyle, alkoxycarbonyle, aryloxycarbonyle ou aminocarbonyle.

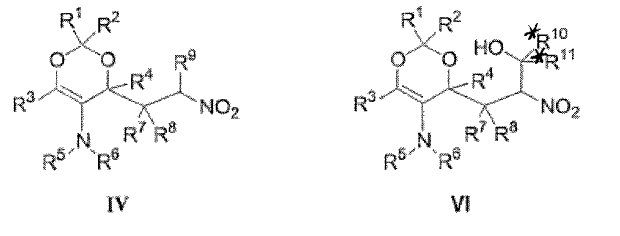

7. Procédé de préparation de composés ayant la formule VII **caractérisé par** a) une réaction de conversion du groupement hydroxy d'un composé ayant la formule #VI sur un bon groupement partant tel qu'un atome d'halogène, un groupement SR, -OC(O)R ou -OS(O)ₙR, où n peut avoir comme valeur 1 ou 2 et R peut être un groupement alkyle linéaire, ramifié ou cyclique ou un groupement aryle, lesquels groupements peuvent à leur tour être remplacés par un ou plusieurs substituts identiques ou différents choisis parmi les atomes d'halogène, les groupements alkyle, aryle, hydroxy, alkyloxy, acyloxy, aryloxy, amino, alkylamino, arylamino suivi de b) l'élimination du dit groupement partant et où les groupements R¹, R², R³, R⁴, R⁵, R⁶, R⁷, R⁸, *R¹⁰ et *R¹¹ peuvent avoir les mêmes valeurs que celles indiquées pour les dits groupes dans la revendication 6.

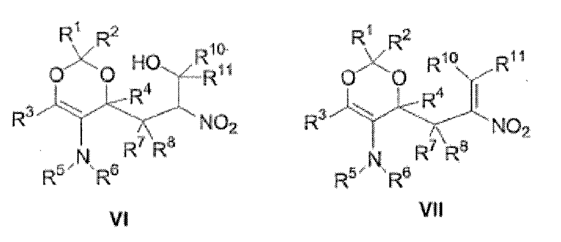

8. Procédé de préparation de composés ayant la formule *V **caractérisé par** la cyclisation d'un composé ayant la formule VII selon la revendication 7, au moyen d'une addition intramoléculaire de type Michael, où les groupements R¹, R², R³, R⁴, R⁵, R⁶, R⁷, R⁸, *R¹⁰ et *R¹¹ peuvent avoir les mêmes valeurs que celles indiquées pour ces dits groupements dans la revendication 6.
